# EUROPÄISCHE PATENTANMELDUNG

(11) **EP 2 033 682 A1**
(43) Veröffentlichungstag der Anmeldung: **11.03.2009**
(21) Anmeldenummer: 07017617.7
(22) Anmeldetag: 07.09.2007
(51) Int. Cl.: A61N 1/05, A61N 1/36, A61F 2/14

(54) **Flexible Sehprothese und ein Verfahren zur Herstellung einer flexiblen Sehprothese**

(71) Anmelder: Fraunhofer-Gesellschaft zur Förderung der angewandten Forschung e.V., 80686 München (DE)
(72) Erfinder: Goertz, Michael, 47608 Geldern (DE); Trieu, Khiem, Dr. Hoc., 47475 Kamp-Lintfort (DE); Mokwa, Wilfried, Prof. Dr., 47800 Krefeld (DE)
(74) Vertreter: Zinkler, Franz

(57) **Zusammenfassung**

Eine Sehprothese (100) für ein Auge (101) mit einer Linse (102) und einer Netzhaut (103) weist ein erstes, zweites und drittes Substratteil (110, 120, 130) auf. Der erste Substratteil (110) weist eine Spule (105) auf und ist in der Linse (102) fixierbar. Der zweite Substratteil (120) weist Stimulationselektroden (122) und Verbindungsleitungen (125) auf und ist an der Netzhaut (103) fixierbar. Der dritte Substratteil (130) weist eine Verarbeitungseinheit (132) auf und verbindet das erste und das zweite Substratteil (110, 120) miteinander, wobei die Verarbeitungseinheit (132) elektrisch mit der Spule (105) und mit den Stimulationselektroden (122) verbunden und ausgelegt ist, ein in der Spule (105) durch eine elektromagnetische Welle (156a) erzeugtes Signal in ein Stimulationsimpuls umzuwandeln und an die Stimulationselektroden (122) über die Verbindungsleitung (125) weiterzuleiten. Das erste Substratteil (110) ist bezüglich des dritten Substratteils (130) faltbar.

## Beschreibung

Die vorliegende Erfindung bezieht sich auf eine flexible Sehprothese und ein Verfahren zur Herstellung einer flexiblen Sehprothese und insbesondere auf eine epiretinale Sehprothese, die erblindeten Patienten wieder eine Sehwahrnehmung ermöglichen kann.

Eine epiretinal implantierbare Sehprothese beruht auf einer elektrischen Stimulation von Ganglienzellen bei Patienten, die beispielsweise durch eine Degeneration der vorgeschalteten retinalen Neuronen erblindet sind. Derartige Situationen treten beispielsweise bei Retinitis Pigmentosa (RP) auf, aber auch bei weit fortgeschrittenen Fällen einer Makuladeneration. Aber selbst bei weit fortgeschrittenen Fällen von Retinitis Pigmentosa und trotz einer klinisch oft erkennbaren Atrophie des Sehnervs sind meist noch ausreichend Ganglienzellen vorhanden, die für eine funktionelle Elektrostimulation genutzt beziehungsweise rekrutiert werden können.

Das Konzept epiretinaler Sehprothesen (Implantate, die ins Auge eingepflanzt werden) besteht in einer elektrischen Stimulation von Ganglienzellen und zwar von der Augeninnenseite der Netzhaut her. Hierzu bedarf es einer Matrix bzw. eines Arrays von möglichst kleinen und möglichst zahlreichen Stimulationselektroden, die auf die Netzhautoberfläche gebracht werden. An den Reizelektroden werden gemäß des Seheindrucks Stimulationspulsmuster (elektrische Reize) ins Nervengewebe abgegeben, die zu einer möglichst optimalen Aktivierung der Ganglienzellen führen.

Die Aufnahme der Bilddaten kann beispielsweise mittels einer Kamera (durch einen CMOS-Kamerachip) geschehen, wobei die Kamera beispielsweise in einem Brillenglas integriert werden kann. Von einem Sehprozessor (Verarbeitungseinheit) werden aus diesen Bildern diejenigen Stimulationsimpulse ermittelt, die geeignet sind, die Ganglienzellen so zu aktivieren, dass ein möglichst realitätsnahes Bild entstehen kann. Das Stimulationssignal wird dabei mittels eines externen Senders, der beispielsweise ebenfalls in der Brille integriert sein kann, kodiert, moduliert und zum Implantat übertragen. Nach einer Datenrückgewinnung in einer Empfangselektronik (Empfangsteil + Elektronik), die in dem Implantat (Sehprothese) angeordnet werden, wird durch die Stimulationselektronik elektrodenspezifische, biphasische Impulse erzeugt, die dann die Ganglienzellen über ein biokompatibles Elektrodenarray elektrisch innervieren.

Das Implantat innerhalb des Auges weist mehrere Bestandteile auf, die beispielsweise einen Empfangsteil in einer Kunstlinse und einen Stimulator auf der Netzhaut umfassen kann. Energie und Daten für das Implantat werden drahtlos auf elektromagnetischem Wege (z.B. mittels Radiowellen) von dem Sender außerhalb des Auges zu dem Empfangsteil übertragen und von dort, nach einer Datenrückgewinnung über eine Mikrokabelverbindung zum Stimulator weitergeleitet. Der Stimulator kann beispielsweise die Stimulationselektroden und eine Stimulationselektronik aufweisen, die die Information, die von der Empfangselektronik kommt beziehungsweise von der Empfangselektronik empfangen wurde, umsetzt.

Bei bisherigen Sehprothesen wurde auf eine dünne flexible Kunststofffolie die Elektronikkomponenten, ein Empfangschip, ein Stimulatorchip, SMD-Bauteile (SMD = surface mounted device) und eine handgewickelte Empfangsspule montiert und anschließend mit Silikon verkapselt. Der Empfangsteil ist dabei als Kunstlinse ausgeführt und der Stimulatorchip ist in der Nähe der Stimulationselektroden angeordnet. Um die Bioverträglichkeit des Systems herzustellen, wurden die Komponenten von einem Hüllkörper aus Silikon umgeben, der nur die Stimulationselektroden freilässt.

Um ein solches konventionelle System in ein Auge einzubringen, wird zunächst die Augenlinse über eine Öffnung in der Hornhaut entfernt (vergleichbar zur Kataraktoperation). Im Anschluss daran wird der Glaskörper entfernt und zum Einbringen des Stimulationsteils wird der Kapselsack geöffnet. Der Stimulatorteil wird dann über die Hornhautöffnung und die Kapselsacköffnung in das Augeninnere geschoben. Daran anschließend wird die Kunstlinse in den Kapselsack platziert. Wegen der Größe der eingesetzten elektronischen Komponenten sind sehr große Öffnungen erforderlich, was das Risiko bei der Operation stark erhöht.

Bei einer Weiterentwicklung konnten diese konventionellen Systeme miniaturisiert werden. Die Spule dieses weiterentwickelten Systems ist nicht mehr handgewickelt, sondern mit Hilfe einer Mikrogalvanik auf einen flexiblen Träger aufgebracht. Die Chips befinden sich ferner außerhalb der Linse und es ist deshalb möglich, die Kunstlinse zu falten. Zur Montage der Chips wird die Flip-Chip-Technologie eingesetzt und durch die Verwendung der Mikrospule wurde es somit möglich, den Bereich der Kunstlinse zu falten. Dies führte zu einer deutlichen Reduzierung der zum Einbringen der Kunstlinse erforderlichen Öffnung.

Die weiterentwickelten Systeme sind jedoch dahingehend nachteilig, dass sich sowohl die SMD-Bauteile als auch die beiden Chips auf den Kabelteil befinden, der die Spule mit den Stimulationselektroden verbindet. Durch die Starrheit dieser Bauteile ist die Flexibilität in dem Bereich, wo die Bauteile und Chips sich befinden, jedoch stark reduziert. Dies erschwert erheblich die Implantation dieser weiterentwickelten Systeme. Durch die Aufbauhöhe der einzelnen Komponenten wird weiterhin eine relativ große Öffnung im Kapselsack erforderlich sein, um das Implantat ins Auge einzuführen.

Ferner lassen sich die Stimulationselektroden aufgrund des Gewichts der Bauteile (SMD-Bauteile, Chips) nur sehr schwer genau platzieren und es ist somit eine zusätzliche Fixierung der Folie im Bereich der elektronischen Bauteile erforderlich.

Ausgehend von diesem Stand der Technik liegt der vorliegenden Erfindung die Aufgabe zugrunde, eine Sehprothese beziehungsweise ein Implantat zu schaffen, welches zum Einen leicht ins Auge implantierbar ist und zum anderen eine stabile Fixierung im Auge ermöglicht.

Diese Aufgabe wird durch eine Sehprothese gemäß Anspruch 1 und Anspruch 26, ein Verfahren zur Verwendung nach Anspruch 27 und ein Verfahren zum Herstellung gemäß Anspruch 28 gelöst.

Der vorliegenden Erfindung liegt die Erkenntnis zugrunde, dass eine Sehprothese für ein Auge mit einer Linse und einer Netzhaut ein Substrat mit einem ersten, zweiten und dritten Substratteil aufweist, wobei der erste Substratteil eine Spule aufweist und in der Linse fixierbar ist, der zweite Substratteil Stimulationselektroden und eine Verbindungsleitung aufweist und an der Netzhaut fixierbar ist, der dritte Substratteil eine Verarbeitungseinheit aufweist und den ersten und den zweiten Substratteil derart verbindet, dass der erste bezüglich des dritten Substratteils faltbar ist. Ferner ist die Verarbeitungseinheit elektrisch mit der Spule und mit den Stimulationselektroden verbunden und ausgelegt, um ein in der Spule durch eine elektromagnetische Welle erzeugtes Signal in Stimulationsimpulse umzuwandeln und die Stimulationsimpulse an die Stimulationselektroden weiterzuleiten.

Bei Ausführungsbeispielen der vorliegenden Erfindung kann die Faltung derart ausgeführt, dass das dritte Substratteil mit der Verarbeitungseinheit zusammen mit dem ersten Substratteil in der Linse fixierbar ist und die Verbindungsleitungen eine elektrische Verbindung zu dem zweiten Substratteil und somit zu dem Stimulationselektroden herstellt. Dazu kann der erste Substratteil vorzugsweise eine flexible Folie in Form einer Kreisscheibe oder eines Kreisringes mit einem äußeren und inneren Kreisrand aufweisen, wobei und die Spule entlang der Kreisscheibe gewickelt ist. Damit kann beispielsweise der erste Substratteil, wenn er auf den dritten Substratteil gefaltet ist, noch einmal gefaltet werden, so dass beim Implantieren nur ein kleiner Schlitz im Auge (oder der Linse) erforderlich ist. Die zweite Faltung kann dabei beispielsweise parallel zu einer Implantationsrichtung oder parallel zu der Verbindungsleitung erfolgen. Somit kann die Sehprothese noch vor dem Implantieren zweifach gefaltet werden, was die Implantation deutlich vereinfacht.

Weitere Ausführungsbeispiele weisen lösbare Elektrodenbefestigungen und Positionierungshilfen auf, die ein leichtes Fixieren und Lösen des Stimulators unterstützen. Beispielsweise können die Elektrodenbefestigungen seitlich Öffnungen aufweisen, so dass ein Lösen des Stimulators von den Retinanägeln oder -stiften durch ein einfaches Ausschieben des Stimulators (ohne die Retinanägel zu ziehen) erfolgen kann. Umgekehrt kann ebenfalls ein leichtes Befestigen durch ein einfaches Hineinschieben erfolgen. Ferner können die Elektrodenbefestigungen ebenfalls ein Längsloch aufweisen, so dass der Stimulator ebenfalls durch ein Auf- oder Abschälen an einem Retinanagel befestigt werden kann.

Somit werden mit Ausführungsbeispielen der vorliegenden Erfindung die oben genannten schwerwiegenden Nachteile beseitigt beziehungsweise überwunden. Es wird insbesondere möglich, alle elektronischen Komponenten in dem Bereich der Kunstlinse (oder Linse) zu verlegen, so dass die Flexibilität der Folie von der Linse bis zu den Stimulationselektroden erhalten bleibt. Dies ist insbesondere deshalb möglich, da zwischen den Komponenten, welche in der Linse angeordnet sind, und den Stimulationselektroden, die auf der Netzhaut befestigt werden, nur das Verbindungsteil angeordnet ist und dieses Verbindungsteil eine flexible Folie (mit eingebetteten Leiterbahnen) aufweisen kann.

Die benötigten operativen Öffnungen können dadurch deutlich minimiert werden - insbesondere dann, wenn die Folie im Bereich der Kunstlinse (bzw. der Bauelemente) beispielsweise sichelförmig erweitert wird oder einen sichelförmigen Teil aufweist und mit Leiterbahnen zur elektrischen Kontaktierung der elektronischen Bauelemente versehen wird. Nach einer Kontaktierung und Fixierung der Bauelemente kann die Spule dann um 180 Grad auf den sichelförmigen Teil mit den elektronischen Bauteilen gefaltet werden. Der sichelartige Abschnitt sollte dabei derart ausgeführt werden, dass die runde Form der Folie im Bereich der Kunstlinse nach dem Falten erhalten bleibt. Der Zwischenraum zwischen der Spule und den Komponenten kann mit geeignetem Klebstoff aufgefüllt werden, so dass nach der Faltung die Folie mit der Spule (erstes Substratteil) und der sichelförmige Teil (dritte Substratteil) aufeinander kleben. Vorzugsweise wird die Faltung dabei derart ausgeführt, dass nach der Faltung der Innenraum innerhalb des inneren Kreisrandes des ersten Substratteils freigelassen wird. Das kann durch eine geeignete Gestaltung des sichelförmigen Teils erreicht werden. Im Anschluss an die Faltung und das optionale Verkleben kann der gefaltete erste und dritte Substratteil auch mit Silikon (oder einem anderen Material) vergossen werden, so dass eine Verkapselung entsteht.

Ausführungsbeispiele der vorliegenden Erfindung besitzen somit eine Reihe von Vorteilen gegenüber den zuvor beschriebenen konventionellen Implantaten. Durch die sichelförmige Anordnung wird zum einen die zentrale Öffnung des ersten Substratteils freigelassen, so dass eine visuelle Beobachtung der Netzhaut möglich ist. Ferner kann durch die sichelförmige Gestalt des dritten Substratteil erreicht werden, dass im gefalteten Zustand immer noch eine Hälfte der als Kreisscheibe geformte erste Substratteil faltbar bleibt, d.h. es bleibt durch die so erreichte halbkreisförmige Anordnung die Linse zur Hälfte faltbar. Ferner ist es möglich, wenn weiterer Platz für elektronische Komponenten benötigt wird, weitere Sicheln hinzuzufügen, die dann ebenso aufeinander gefaltet werden können, so dass weitere Substratteile übereinander gefaltet werden können. Ein weiterer Vorteil dieser Anordnung besteht darin, dass alle Komponenten inkl. der Spule an beiden Seiten durch die flexible Folie geschützt werden können und mit einem Klebstoff vergossen werden. Somit kann beispielsweise die Wirksamkeit der Verkapselung hinsichtlich eines effektiven mechanischen Schutzes als auch hinsichtlich eines Eindringens von Feuchtigkeit, wesentlich erhöht werden.

Vorteilhaft ist ebenfalls, dass die Faltkante beispielsweise dadurch vorgegeben werden oder erleichtert werden kann, dass Einschnürungen in der Folie in dem Bereich des Übergangs in dem Spulenbereich (erster Substratteil) und dem Bereich zum Übergang zu den Elektrodenleitungen (Verbindungsleitungen) vorgesehen werden. Ferner ist es möglich, durch Anbringen entsprechender Justiermarken im Spulenteil als auch in dem sichelförmigen Teil (zweiter Substratteil) eine genaue und reproduzierbare Faltung zu ermöglichen. Durch ein Abknicken des Verbindungsteils zwischen der Kunstlinse und dem Stimulationselektrodenarray (Stimulationselektroden) kann ferner ein beliebiger Winkel zwischen der Kunstlinse und den abgehenden Leitungen eingestellt werden. Dies ist möglich, da der Verbindungsteil flexibel gestaltet ist. Ferner erleichtert dies das Platzieren des Elektrodenarrays auf der Netzhaut.

Schließlich ist die erfindungsgemäße Ausgestaltung der Elektrodenbefestigung mit Positionierungshilfen dahingehend vorteilhaft, dass die Retinanägel oder -stifte bei einem evtl. notwendigen Explantieren der Sehprothese nicht entfernt werden brauchen und eine einfaches Rein- und Rausschieben des Stimulators ermöglicht wird. Die Kombination der Elektrodenbefestigung mit den Positionierungshilfen ist fern dahingehend vorteilhaft, dass somit die Position des Stimulators unterhalb der Retinanägel genau bestimmbar ist.

Ausführungsbeispiele der vorliegenden Erfindung werden nachfolgend bezugnehmend auf die beiliegenden Zeichnungen näher erläutert. Es zeigen:
- Fig. 1: eine Sehprothese gemäß einem Ausführungsbeispiel der vorliegenden Erfindung;
- Fig. 2: die Sehprothese nach einer Faltung;
- Fig. 3: ein Systemkonzept der epiretinalen Sehprothese;
- Fig. 4A,B: eine Draufsicht und eine Schnittdarstellung einer Faltungsunterstützungseinrichtung;
- Fig. 5: eine mechanische Justierhilfe gemäß einem Ausführungsbeispiel der vorliegenden Erfindung;
- Fig. 6: eine Justage eines Retinanagels unter Verwendung der Positionierungshilfe;
- Fig. 7: eine Querschnittsansicht einer dreidimensionalen Elektrodenstruktur .

Bezüglich der nachfolgenden Beschreibung sollte beachtet werden, dass bei den unterschiedlichen Ausführungsbeispielen gleiche oder gleichwirkende Funktionselemente gleiche Bezugszeichen aufweisen und dass eine wiederholte Beschreibung dieser Elemente weggelassen wird.

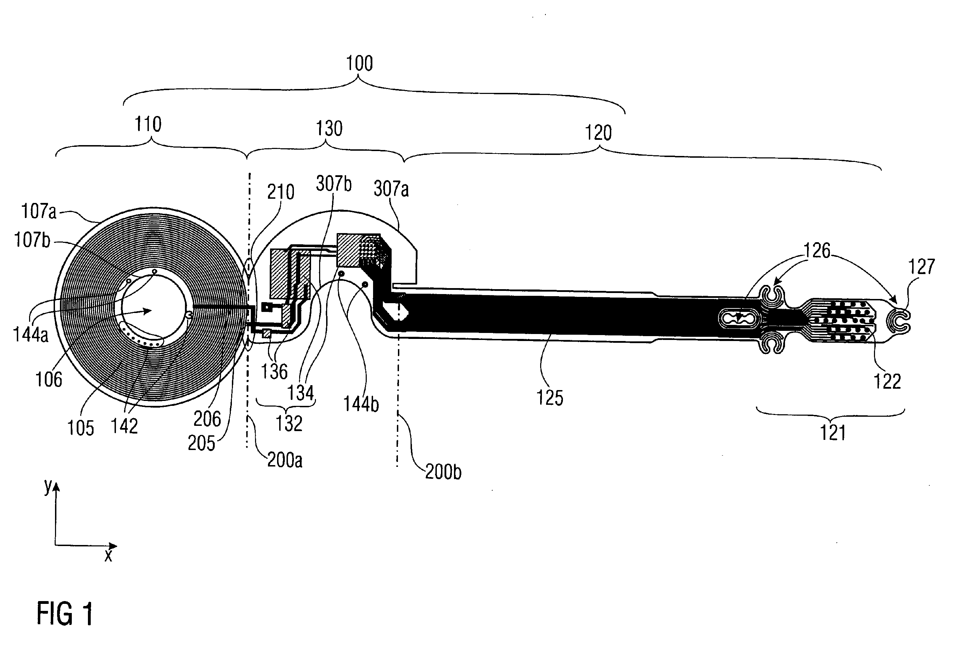

Fig. 1 zeigt eine Sehprothese 100 oder ein Implantat gemäß einem Ausführungsbeispiel der vorliegenden Erfindung. Die Sehprothese 100 weist einen ersten Substratteil 110 und einen zweiten Substratteil 120 auf, die durch einen dritten Substratteil 130 miteinander verbunden sind.

Der erste Substratteil 110 weist eine Spule 105 auf, die scheibenförmig als Kreisring ausgestaltet ist und einen zentralen Bereich 106 freilässt. Leitungen der Spule 105, die von einer ersten und zweiten Verbindungsleitung 205, 206 kontaktiert werden, sind somit kreisförmig zwischen einem äußeren Kreisrand 107a und einem inneren Kreisrand 107b, der den zentralen Bereich 106 offen lässt, herumgewickelt.

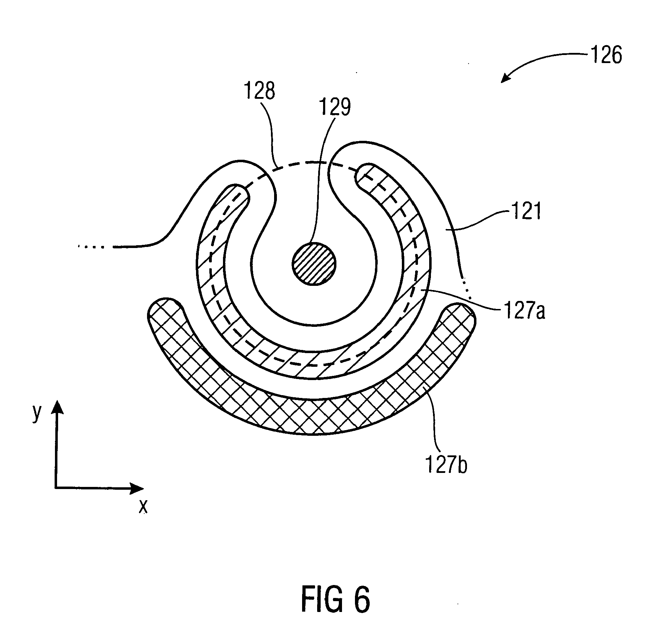

Der zweite Substratteil 120 weist einen Stimulator 121 mit Stimulationselektroden 122, die mit Verbindungsleitungen 125 kontaktiert werden, auf. Der Stimulator 121 mit den Stimulationselektroden 122 dient einer Stimulation der Netzhaut (Retina) oder genauer der Ganglienzellen und kann mit den Befestigungshilfen 126 (Elektrodenbefestigung) an der Netzhaut befestigt werden. Zur besseren Orientierung oder zur Justage weisen die Elektrodenbefestigung 126 Positionshilfen 127 auf (siehe Fig. 6 unten). Der dritte Substratteil 130 weist eine Verarbeitungseinheit 132 auf, die beispielsweise SMD-Komponenten 136 und Halbleiterschaltungen 134 aufweisen kann.

Der dritte Substratteil 130 ist vorzugsweise sichelförmig ausgestaltet, so dass der dritte Substratteil 130 zwei halbkreisförmige Ränder - einen äußeren Sichelkreis 307a und einen inneren Sichelkreis 307b - aufweist. Die Verarbeitungseinheit 132 ist entlang der Sichel angeordnet und erzeugt Stimulationssignale für die einzelnen Stimulationselektroden 122. Der dritte Substratteil 130 ist mit dem ersten Substratteil 110 entlang einer ersten Trennlinie 200a und mit dem zweiten Substratteil 120 entlang einer zweiten Trennlinie (oder weiteren Trennlinie) 200b miteinander verbunden, wobei in Fig. 1 der erste bis dritte Substratteil 110, 120, 130 in einer (x,y)-Ebene entlang der x-Richtung derart angeordnet sind, so dass die erste und zweite Trennlinie 200a und 200b parallel zur y-Achse verlaufen. Bei weiteren Ausführungsbeispielen können jedoch die Trennlinien 200 anders ausgerichtet sein. Entlang der ersten Trennlinie 200a ist eine Faltunterstützungseinrichtung 210 ausgebildet, um die Faltung entlang der ersten Trennlinie 200a zu unterstützen.

Zur besseren Justierung des ersten und des dritten Substratteils 110, 130 nach einer Faltung kann optional eine erste Justagehilfe (Justiermarke) 144a, die mehrere Komponenten aufweisen kann, an dem ersten Substratteil 110 und eine zweite Justagehilfen 144b, die ebenfalls mehrere Komponenten aufweisen kann, an dem dritten Substratteil 130 ausgebildet sein. Die Justagehilfe 144 ist dabei derart ausgebildet, dass nach der Faltung die erste Justagehilfe 144a und die zweite Justagehilfe 144b in Übereinstimmung gebracht werden, und in ähnlicher Art und Weise, sofern vorhanden, weitere Komponenten ebenfalls in Übereinstimmung gebracht werden. Die erste Justagehilfen 144a als auch eine Implantatbeschriftung und Codierung 142 kann beispielsweise entlang des inneren Kreisrandes 107b angeordnet sein.

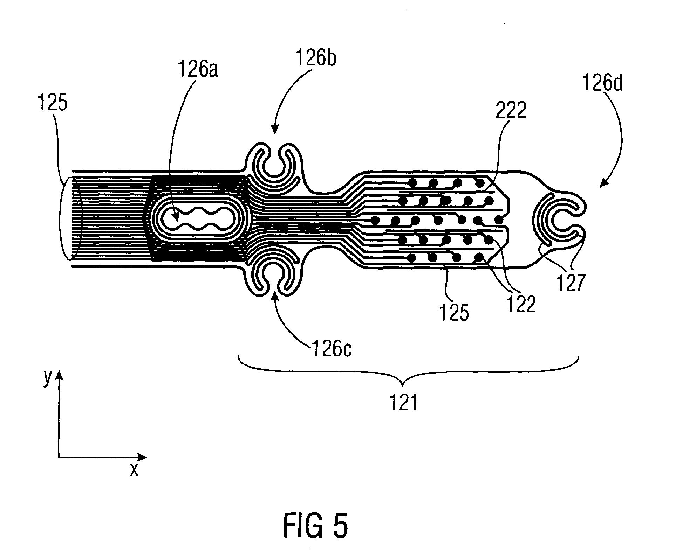

Die Verbindung zwischen dem dritten Substratteil 130 und dem zweiten Substratteil 120 entlang der zweiten Trennlinie 200b ist derart ausgestaltet, dass das zweite Substratteil 120 und das dritte Substratteil 130 entlang der zweiten Trennlinie 200b faltbar sind. Die Verbindungsleitungen 125, die eine Kontaktierung der Stimulationselektroden 122 realisieren (Verbindung zwischen der Verarbeitungseinheit 132 und den Stimulationselektroden 122) können beispielsweise galvanisch verstärkt sein, so dass ein elektrischer Widerstand möglichst niedrig gehalten werden kann. Ferner kann die Elektrodenbefestigung 126 in einer lösbaren Form ausgestaltet sein, was weiter unten in Fig. 5 detaillierter beschrieben wird.

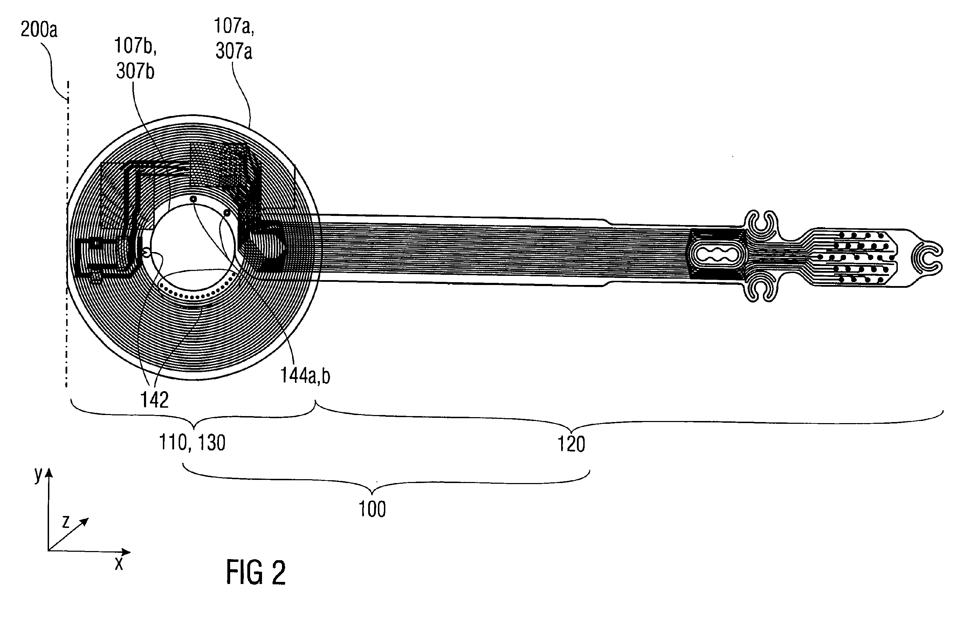

Fig. 2 zeigt das Resultat der Faltung des ersten Substratteils 110 bezüglich des dritten Substratteils 130 entlang der ersten Trennlinie 200a. Da der dritte Substratteil 130 vorzugsweise, wie in Fig. 1 gezeigt, eine sichelförmige Gestalt aufweist, kann der äußere Sichelkreis 307a derart geformt sein, dass nach einer Faltung entlang der ersten Trennlinie 200a der äußere Sichelkreis 307a mit dem äußeren Kreisrand 107a in Übereinstimmung gebracht werden kann. In ähnlicher Art und Weise kann der innere Sichelkreis 307b bevorzugt derart gestaltet sein, dass nach einer Faltung entlang der ersten Trennlinie 200a der innere Sichelkreis 307b mit dem inneren Kreisrand 107b in Übereinstimmung gebracht werden kann.

Somit kann infolge der sichelförmige Gestalt des dritten Substratteils 130 bei einer Faltung entlang der ersten Trennlinie 200a der dritte Substratteil 130 durch den ersten Substratteil 110 abgedeckt werden, so dass insbesondere der zentrale Bereich 106 frei bleibt.

Für eine korrekte Justierung des ersten und dritten Substratteils 110, 130 bei der Faltung können die Justagehilfen 144a und 144b genutzt werden. Beispielsweise kann die erste Justagehilfe 114a als ein Rand einer geometrischen Figur (z.B. Kreis, Viereck, etc.) und die zweite Justagehilfe 114b kann beispielsweise als die geometrische Figur ausgestaltet sein. Somit ist es leicht möglich, z.B. einen Kreis (erste Justagehilfe 144a) mit einer Kreisscheibe (zweite Justagehilfe 144b) in Übereinstimmung zu bringen. Vorzugsweise weist der Kreis einen größeren Radius auf als die Kreisscheibe (die Kreisscheibe kann z.B. auch ein Punkt sein), was eine Zentrierung der Kreisscheibe innerhalb des Kreises erleichtert. Das gleiche Prinzip kann ebenfalls auf andere geometrische Figuren angewendet werden.

Es ist ferner vorteilhaft, wenn der dritte Substratteil 130 entlang des inneren Sichelkreises 307b transparent ist, so dass die Implantatbeschriftung und Codierung 142 nach der Faltung des dritten Substratteils 130 bzgl. des ersten Substratteils 110 sichtbar sind. Eine andere Möglichkeit ist es, die Implantatbeschriftung und Codierung 142 in einem Randbereich entlang des inneren Kreisrandes 107b anzuordnen, der nach der Faltung des dritten Substratteils 130 immer noch frei liegt, z.B. in jenem Teil entlang des inneren Kreisrandes 107b angeordnet sind, der nicht mit dem inneren Sichelkreises 307b bei der Faltung entlang der ersten Trennlinie 200a zusammengebracht wird. Wie in Fig. 2 gezeigt, sind dadurch die Implantatbeschriftung und Codierung 142 noch klar erfassbar.

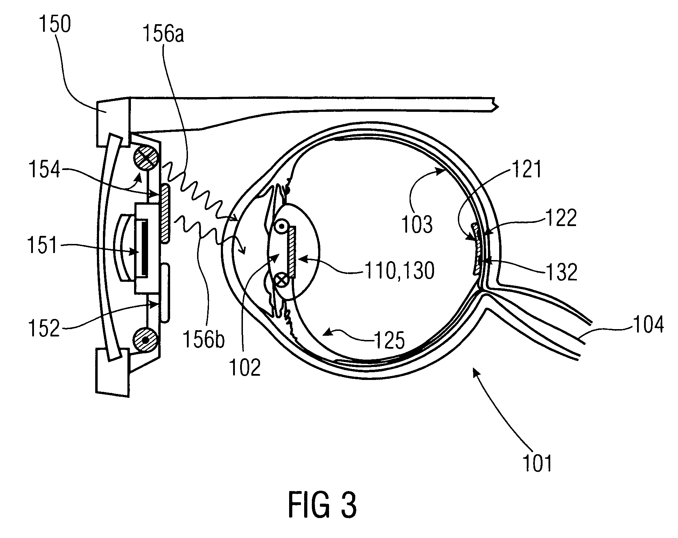

Fig. 3 zeigt ein Systemkonzept für eine epiretinale Sehprothese 100, wie sie beispielsweise in Fig. 1 beschrieben wurde. Es ist ein Auge 101 gezeigt, das eine Linse 102, eine Netzhaut oder Retina 103 und einen Sehnerv 104 aufweist. Die Linse kann auch als Kunstlinse ausgestaltet sein. Die Sehprothese 100 wird derart in das Auge transplantiert, so dass der erste und dritte Substratteil 110 und 130 in der Linse 102 fixiert werden und die Verbindungsleitungen 125 einen Kontakt zu dem Stimulator 121 herstellt, wobei der Stimulator 121 (oder Retinastimulator) an der Retina beispielsweise in jenem Bereich befestigt werden kann, wo bei einem gesunden Auge ein Bild erzeugt wird. Um ein Bild durch Stimulation der Retina erzeugen zu können, bedarf es zunächst einer Bilderzeugung (z.B. durch einen Stimulationsmusterencoder), was beispielsweise mittels einer speziellen Brille 150 geschehen kann. Die Brille 150 weist dabei beispielsweise zunächst eine Kamera 151 auf, die beispielsweise einen Single-Chip-CMOS-Kamera umfassen kann. Ferner weist die Brille einen Signalprozessor 152 auf, der beispielsweise die von der Kamera 151 erzeugten elektronischen Bilddaten entsprechend verarbeitet (z. B. kodiert und moduliert). Die so erhaltenen Daten können dann von einem Datensender 154 (Telemetrietransmitter) an die Sehprothese 100 übertragen werden. Die Übertragung kann beispielsweise mittels einer elektromagnetischen Welle 156a, aber auch optisch 156b geschehen. Die Frequenz der elektromagnetischen Welle 156a zur Datenübertragung kann beispielsweise in einem Bereich zwischen 0,1 und 100 MHz oder zwischen 10 und 15 MHz liegen oder ungefähr 13,56 MHz betragen. Die elektromagnetische Welle 156a wird von der Spule 105 in dem ersten Substratteil 110 empfangen und in ein elektrischen Impuls umgewandelt, der dann von der Verarbeitungseinheit 132 ausgewertet werden.

Die elektromagnetische Welle 156a kann neben den Bilddaten, die von der Kamera 151 erzeugt wurden, auch die zum Betrieb der Sehprothese 100 nötige Energie übertragen. Dies kann beispielsweise in ähnlicher Art und Weise geschehen, wie es in RFID-Systemen (RFID = radio frequency identification) üblich ist. Die Spule 105 in dem ersten Substratteil 110 dient somit als Telemetriereceiver sowohl dem Empfang von Daten als auch der Energieversorgung (Empfang der Versorgungsenergie) und ist beispielsweise in der Linse 102 fixierbar. Die empfangenen Daten werden anschließend über die Verbindungsleitungen 125, die beispielsweise Mikrokabel aufweisen können, an den Stimulator 121 übermittelt, wobei der Stimulator 121 einen Array von Stimulationselektroden 122 und optional eine Stimulatorelektronik aufweisen kann. Der Stimulator 121 kann eine entsprechende Verkapselung aufweisen, die beispielsweise einen Schutz vor der entlang der Netzhaut vorhanden Flüssigkeit bietet. Nach der Stimulation der Retina 103 durch die elektrischen Impulse, die an dem Stimulator 121 anliegen und entsprechend den Bilddaten, wie sie durch die Kamera 151 erfasst wurden, erzeugt wurden, kann der Sehnerv 104 entsprechende Impulse an das Gehirn weiterleiten und somit eine Sehwahrnehmung auslösen.

Somit umfassen Ausführungsbeispiele der vorliegenden Erfindung neben der Sehprothese 100 noch einen Stimulationsmustergenerator, der beispielsweise in der Brille 150 angeordnet sein kann, und eine Telemetrie zur Datenübertragung und zur Energieversorgung aufweist. Die Datenübertragung kann ebenfalls optisch erfolgen. In diesem Fall benötigt die Sehprothese 100 ebenfalls eine Einrichtung zum Empfangen von optischen Daten, beispielsweise mittels eines optischen Sensors, der ausgebildet ist, um die durch die Sendeeinrichtung 154 ausgesandten optischen Signale zu empfangen und zu verarbeiten. Die optische Signalübertragung kann dabei beispielsweise mittels eines speziellen Lasers (z. B. im Infratorbereich) geschehen.

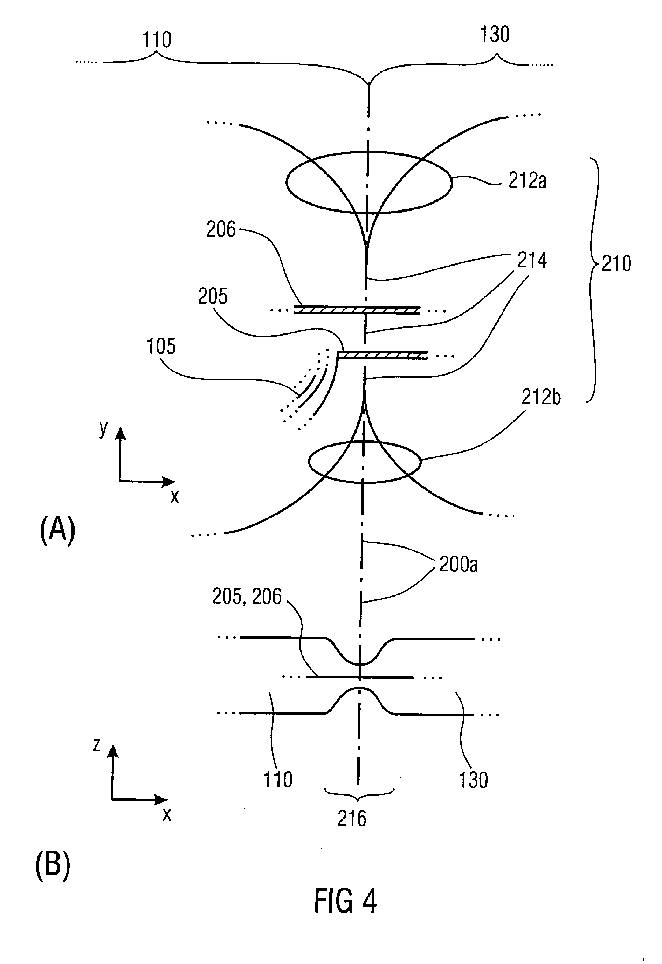

Fig. 4A,B zeigen ein Ausführungsbeispiel für die Faltenunterstützungseinrichtung 210, die beispielsweise entlang der ersten Trennlinie 200a ausgebildet ist. Die erste Trennlinie 200a trennt dabei das erste Substratteil 110 von dem dritten Substratteil 130 (auf der rechten Seite in der Fig. 4).

Fig. 4A zeigt eine Draufsicht auf diesen Abschnitt der Sehprothese 100, der in der (x,y)-Ebene angeordnet ist. Dieser Bildausschnitt zeigt ebenfalls einen Ausschnitt aus der Spule 105, die durch die erste Verbindungsleitung 205 kontaktiert wird, wobei die zweite Verbindungsleitung 206 die Spule 105 von dem anderen Ende her (in der Fig. 4 nicht gezeigt) kontaktiert (siehe Fig. 1). Die Faltenunterstützungseinrichtung 210 weist zum einen eine erste und zweite Einkerbung 212a, 212b in dem Übergangsbereich des Substrats von dem ersten Substratteil 110 zu dem dritten Substratteil 130 auf. Ferner kann die Faltenunterstützungseinrichtung 210 Perforationen 214 aufweisen, so dass das Substrat Löcher beziehungsweise Gräben derart aufweist, um eine Faltung entlang der ersten Trennlinie 200a zu erleichtern.

In Fig. 4B zeigt denselben Bildausschnitt, jedoch gedreht in der (x,z)-Ebene, so dass ein Querschnitt durch die Sehprothese 100 parallel zur x-Achse gezeigt ist. Wiederum trennt die erste Trennlinie 200a den ersten Substratteil 110 von dem dritten Substratteil 130. Die Faltunterstützungseinrichtung 210 weist in dem Übergangsbereich zwischen dem ersten Substratteil 110 und dem dritten Substratteil 130 eine Einschnürung 216 auf, so dass das Substrat nahe der Einschnürung 216 eine geringere Schichtdicke aufweist, und somit eine Faltung entlang der ersten Trennlinie 200a erleichtert wird.

Bei weiteren Ausführungsbeispielen ist die Faltenunterstützungseinrichtung 210 anders ausgebildet oder nur Teile der in Fig. 4 gezeigten Möglichkeiten, die Einkerbung 212a, 212b, die Perforationen 214 und die Einschnürung 216, sind realisiert. Die Faltenunterstützungseinrichtung 210 ist derart ausgebildet, so dass die erste und zweite Verbindungsleitung 205, 206 bzw. weitere Verbindungsleitungen, die in der Fig. 4 nicht gezeigt sind, durch die Faltenunterstützungseinrichtung 210 nicht beschädigt oder unterbrochen werden. Beispielsweise sind die erste und zweite Verbindungsleitung 205, 206 derart ausgebildet, dass nach der Faltung ein einwandfreier Stromfluss beziehungsweise eine einwandfreie Signalübertragung gewährleistet werden kann.

Fig. 5 zeigt den Retina-Stimulator 121, der durch die Verbindungsleitungen 125 kontaktiert wird. Der Retina-Stimulator 121 weist beispielsweise die Stimulationselektroden 122 auf, die beispielsweise einzeln durch jeweils eine der Verbindungsleitungen 125 kontaktiert werden und arrayförmig in dem Stimulator 121 angeordnet sein können. In dem gezeigten Ausführungsbeispiel der Fig. 5 ist das Array der Stimulationselektroden 122 derart ausgebildet, dass in fünf Reihen, eine erste Reihe vier Elektroden, eine zweite Reihe fünf Elektroden, eine dritte Reihe sieben Elektroden, eine vierte Reihe fünf Elektroden und eine fünfte Reihe vier Elektroden aufweist. Die einzelnen Reihen des Arrays können optional durch Trennwände 222 voneinander getrennt sein. Die Trennwände 222 können beispielsweise eine abschirmende Wirkung aufweisen, so dass elektrische Stimulationen eines ersten Bereiches begrenzt werden und eine weitere elektrische Stimulation eines zweiten Bereiches durch die Trennwände 222 unterdrückt wird. Somit kann beispielsweise eine verbesserte Isolierung/Trennung der durch die Stimulationselektroden 122 elektrisch stimulierten Retinaregionen erreicht werden.

In Fig. 5 ist beispielhaft nur eine Form für den Array gezeigt und bei weiteren Ausführungsbeispielen kann das Array auch eine andere Form aufweisen (beispielsweise viereckig oder rechteckig oder auch rund angeordnet sein) oder die Anzahl der Stimulationselektroden 122 kann ebenfalls variiert werden. Ferner kann bei weiteren Ausführungsbeispielen der mittlere laterale Abstand der Stimulationselektroden 122 entlang der x-Richtung variabel sein.

Zur Fixierung des Stimulators 121 dient die Elektrodenbefestigung 126, die verschiedene Formen aufweisen kann. Beispielsweise kann eine erste Elektrodenbefestigung 126a länglich oder schlitzförmig als Längsloch ausgestaltet sein, so dass eine Verschiebung des Stimulators 121 parallel zur X-Achse immer noch möglich ist - zumindest innerhalb der durch die längliche Ausdehnung vorgegebenen Grenzen. Eine zweite Elektrodenbefestigung 126b kann beispielsweise derart geformt sein, dass ein Hineinschieben des Stimulators 121 entlang der positiven y-Achse unter einem Stift oder Nagel gewährleistet ist. In ähnlicher Art und Weise ist eine dritte Elektrodenbefestigung 126c ausgestaltet, jedoch mit dem Unterschied, dass hier ein Hinunterschieben des Stimulators 121 unter einem Stift oder einem Nagel in negativer y-Richtung ermöglicht wird. Eine vierte Elektrodenbefestigung 126d ist wiederum halbkreisförmig ausgestaltet, mit einer Öffnung entlang der positiven x-Achse, so dass ein Einschieben oder Herunterschieben des Stimulators 121 unter einen Nagel oder Stift entlang der positiven x-Achse ermöglicht wird. Somit sind die zweite, dritte und vierte Elektrodenbefestigungen 126b, 126c, 126d alle kreisförmig ausgestaltet, jedoch mit Öffnungen entlang verschiedener Richtungen. Somit ist ein Heraus- oder Hineinschieben des Stimulators 121 ohne ein Entfernen der Stifte oder Nägel möglich. Dies wird insbesondere dadurch unterstützt, dass der Stimulator 121 wie auch die Verbindungsleitung 125 vorzugsweise ein flexibles Substrat (als Folie) aufweisen.

Fig. 6 zeigt eine Elektrodenbefestigung 126, die eine Positionierungshilfe 127 aufweist, wobei die Positionierungshilfe 127 der Positionierung des Stimulators 121 bezüglich eines Retinanagel 129 mit einem Nagelkopf 128 dient. Die Elektrodenbefestigung 126 ist bei diesem Ausführungsbeispiel kreisförmig ausgestaltet, wobei die Elektrodenbefestigung 126 eine Öffnung entlang der positiven y-Achse aufweist, so dass ein Hinein- und Hinausschieben unterhalb eines Nagelkopfes 128 entlang der positiven und negativen y-Achse ermöglicht wird. Die optimale Position des Stimulators 121 kann beispielsweise dadurch gegeben sein, dass der Nagelkopf 128 beziehungsweise deren Berandungskurve (gestrichelte Linie in der Fig. 6) sich möglichst mittig in der Positionierungshilfe 127 befindet. Dies kann beispielsweise jenem Fall entsprechen, wenn der Nagel oder Stift 129 sich möglichst zentral innerhalb der kreisförmig gestalteten Elektrodenbefestigung 126 befindet.

Die Positionierungshilfe 127 kann ferner mehrere Teile aufweisen, wobei ein erster Teil 127a für eine erste Position des Nagels 129 und der zweite Teil 127b für eine zweite Position des Nagels 129 genutzt werden kann. In beiden Fällen kann beispielsweise die erste und die zweite Position dann erreicht sein, wenn der Nagelkopf 128 beziehungsweise deren äußeren Begrenzungslinie sich mittig oder an einer fest vorgegeben Position bezüglich des ersten und des zweiten Teils 127a und 127b der Positionierungshilfen 127 befindet. Die Teile der Positionierungshilfen 127 können natürlich ebenfalls dazu genutzt werden, um den Stimulator 121 an weiteren Positionen zu fixieren, z.B. wenn die Begrenzungslinie nicht mittig ist, sondern sich an einer anderen bestimmten Position befindet.

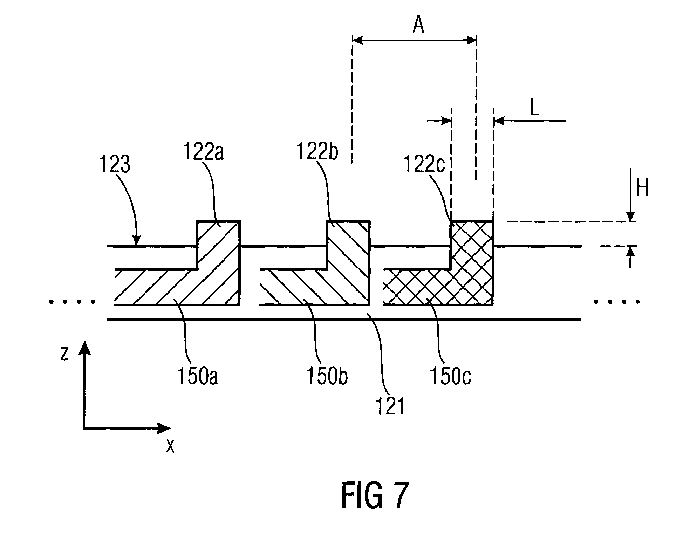

Fig. 7 zeigt ein Ausführungsbeispiel für eine 3-dimensionale Ausgestaltung der Stimulationselektroden 122 entlang des Stimulators 121. Fig. 7 zeigt dazu eine Querschnittsansicht in der (x,z)-Ebene, wobei ein Teil des Stimulators 121 gezeigt ist, der eine erste Stimulationselektrode 122a, eine zweite Stimulationselektrode 122b und eine dritte Stimulationselektrode 122c aufweist. Die erste bis dritte Stimulationselektrode 122a bis 122c sind entlang der X-Richtung angeordnet, wobei die erste Stimulationselektrode 122a durch eine erste Verbindungsleitung 150a, die zweite Stimulationselektrode 122b durch eine zweite Verbindungsleitung 150b und die dritte Stimulationselektrode 122c durch eine dritte Verbindungsleitung 150c kontaktiert werden. Die erste, zweite und dritte Verbindungsleitungen 150a, 150b, 150c stehen in keinem direkten elektrischen Kontakt und werden entlang der Y-Richtung (senkrecht zur Zeichenrichtung) entsprechend versetzt nebeneinander weitergeleitet.

Wie das Ausführungsbeispiel in Fig. 7 zeigt, können die Stimulationselektroden 122 dreidimensional ausgebildet sein, wobei sich die dreidimensionale Ausgestaltung darauf bezieht, dass die Stimulationselektroden 122 nicht an der Oberfläche 123 des Stimulators 121 enden, sondern sich darüber hinaus bis auf eine Höhe H über die Oberfläche 123 erstrecken. Ferner weisen die Stimulationselektroden 122 beispielsweise eine mittlere laterale Ausdehnung L entlang der x-Richtung auf und eine weitere laterale Ausdehnung entlang der y-Richtung, die jedoch in der Fig. 7 nicht gezeigt ist. Der Abstand A benachbarter Stimulationselektroden 122 kann variieren und entsprechend angepasst werden.

Bei der dreidimensionalen Ausgestaltung der Stimulationselektroden 122 kann beispielsweise die Höhe H zumindest 10 oder zumindest 50 % der lateralen Ausdehnung L oder der weiteren lateralen Ausdehnung entlang der y-Richtung betragen. Es ist ebenfalls möglich, dass die Höhe H größer gewählt ist, als die laterale Ausdehnung L oder größer gewählt ist als die weitere laterale Ausdehnung oder größer gewählt ist als das Maximum der lateralen Ausdehnung L und der weiteren lateralen Ausdehnung. Die dreidimensional ausgestalteten Stimulationselektroden 122 können beispielsweise auch Gold aufweisen und die Höhe H kann beispielsweise größer als 10 µm oder zumindest 30 µm sein oder in einem Bereich zwischen 30 µm bis 40 µm liegen.

Somit sind bei Ausführungsbeispielen der vorliegenden Erfindung in einer Folie (Substrat) mit dem ersten Substratteil 110, zweiten Substratteil 120 und dritten Substratteil 130 wichtige Funktionalitäten implementierbar, die für einen späteren Einsatz des Implantats (Sehprothese 100) von großer Bedeutung sind. Dies umfasst beispielsweise den Spulenteil (erster Substratteil 110) als auch den sichelförmigen Teil der Folie (dritter Substratteil 130), die beispielsweise Metallstrukturen zur individuellen Codierung der Implantate enthalten können. Durch ein entsprechendes Entfernen von Teilen dieser Metallstrukturen während der Montage der Bauteile kann jedes Implantat mit einer eindeutigen Nummer versehen werden. Die Codierstrukturen können beispielsweise derart platziert sein, dass sie auch nach der Verkapselung und auch noch nach der Implantation erkennbar sind.

Eine digitale Codierung zur Identifikation des Implantats kann beispielsweise derart geschehen, dass auf dem ersten Substratteil Metallpunkte vorgesehen sind (z. B. fünf) und durch ein Entfernen von einzelnen oder einer Gruppe oder allen Punkten eine Codierung vorgenommen werden kann. Bei n Metallpunkten würde das 2ⁿ Kodierungen erlauben (Z.B. bei n=5 sind es 32 Kodierungen). Zusätzlich kann für eine Identifikation noch eine Nummer vorgesehen sein (beispielsweise eine Serien-Nr.). Eine Codierung mittels Entfernen von Punkten ist dahingehend vorteilhaft, da sie von einem Operateur im Moment des Einsetzens vorgenommen werden kann und später Rückschlüsse über den Zeitpunkt beziehungsweise Ort und wer das Implantat eingesetzt hat, zulässt.

Eine weitere Funktionalität ergibt sich daraus, dass der Stimulatorteil 121 bei der Operation über sogenannte Retina-Nägel 129 auf der Netzhaut 103 fixiert wird. Hierzu sind in der Nähe der Stimulationselektroden 122 oder des Stimulationselektrodenarrays Öffnungen (Positionshilfen 126) für diese Nägel 129 vorgesehen. Um eine spätere Explantation des Implantats (Sehprothese 100) zu ermöglichen, sind im mittleren Bereich der Folie Längslöcher 126a und im Randbereich der Folie Löcher mit einer seitlichen Öffnung 126b, 126c, 126d vorgesehen. Die Löcher haben einen Durchmesser in der Größe der Retina-Nagelstifte 129. Durch den Nagelkopf 128 erfolgt eine Fixierung der Folie auf der Netzhaut 103. Durch die Längsgeometrie der Elektrodenbefestigung 126a beziehungsweise die seitliche Öffnung der Löcher (E-lektrodenfixierungen 126b, 126c und 126d) kann bei einer Explantation die Folie über den Nagelkopf 128 abgezogen werden. Der Nagel 129 kann dabei in der Netzhaut 103 verbleiben. Traumatische Schädigungen der Netzhaut durch ein Ziehen des Nagels beziehungsweise der Nägel 129 können dadurch vermieden werden. Die Anzahl der Nägel 129 (ein Nagel oder mehr) kann bei verschiedenen Ausführungsbeispielen variieren.

Eine weitere Funktionalität ist dadurch gegeben, dass der Nagelkopf 128 im Vergleich zum Durchmesser der Öffnung wesentlich größer ist. Dadurch wird bei der Nagelung die Öffnung durch den Nagelkopf 128 verdeckt (siehe Fig. 6), bevor der Nagel oder der Nagelstift 129 die Öffnung erreicht, so dass der Operateur die Lage des Nagelstifts 129 relativ zur Öffnung nicht mehr sehen kann. Die Nagelung erfolgt dann "blind". Um den Operateur eine Orientierungshilfe bieten zu können, kann um den Bereich der Öffnung ein Metallstreifen (Positionshilfen 127), länglich beziehungsweise rund, realisiert werden, dessen Innendurchmesser so groß beziehungsweise etwas größer als der Nagelkopf 128 gewählt sein kann. Diese Struktur bleibt bei der Nagelung immer sichtbar und kann während der Nagelung verwendet werden, um beispielsweise eine genaue Positionierung des Stimulators 121 zu ermöglichen.

Weiterhin ist es möglich, Metallleiterbahnen 125 galvanisch zu verstärken, um den Ohmschen Widerstand deutlich zu verringern und somit eine Wärmeentwicklung innerhalb des Auges auf ein Minimum zu reduzieren.

Außerdem ist es möglich, wie in der Fig. 7 gezeigt, die Stimulationselektroden 122 dreidimensional auszuführen, um im Gegensatz zu planaren Elektroden einen besseren elektrischen Kontakt zu den Ganglienzellen zu bekommen und somit eine bessere elektrische Stimulation der Netzhaut zu ermöglichen. Es ist ferner möglich, ein Material zur Erhöhung der Ladungsübertragungskapazität zu verwenden oder die Stimulationselektroden 122 mit einem solchen Material zu beschichten. Beispielsweise können die Stimulationselektroden 122 mit Metallen wie Iridium, Iridiumoxid oder Platin/Platinschwarz (platin black) beschichtet sein.

Die flexible Trägerfolie (Substrat mit dem ersten bis dritten Substratteil 110, 120, 130) und das Verkapselungsmaterial können beispielsweise durchsichtig gestaltet sein, so dass zusätzlich zur elektromagnetischen Übertragung der Versorgungsenergie und Daten auch eine optische Übertragung möglich wird. Die optische Übertragung kann beispielsweise zur Übertragung von Daten mit einem höheren Datendurchsatz verwendet werden. Beispielsweise kann dazu ein Infrarotlaser mit geringer Energie (oder geringer Wärmeentwicklung) verwendet werden. Bei Nutzung einer optischen Übertragung weist beispielsweise die Kunstlinse beispielsweise einen optischen Empfänger (Photozelle, Photodetektor, etc.) auf.

Somit umfassen Ausführungsbeispiele der vorliegenden Erfindung eine Sehprothese 100, die aus einem flexiblen Substrat mit einer Spule 105, elektronischen Bauelementen 132 und Stimulationselektroden 122 derart aufgebaut ist, dass durch Falten der Spulen 105 auf dem Bereich 130, der die elektronischen Komponenten enthält, diese Komponenten 132 vollständig in dem Bereich der Kunstlinse 102 gelegt werden können.

Bei weiteren Ausführungsbeispielen sind die elektronischen Komponenten 132 halbkreisförmig aufgebaut (sichelförmig), so dass nach dem Falten eine Hälfte der Linse flexibel bleibt. Bei weiteren Ausführungsbeispielen weist die flexible Sehprothese 100 Einschnürungen in der Folie im Bereich des Übergangs zwischen dem Spulenbereich (erster Substratteil 110) und im Bereich zum Übergang auf die Elektrodenleitungen 150 derart auf, dass die Faltrichtung vorgegeben ist und eine Faltung erleichtert wird.

Bei weiteren Ausführungsbeispielen weist der Bereich der Kunstlinse in der Mitte eine Öffnung 106 auf, so dass der Operateur durch diese Öffnung ins Augeninnere schauen kann. Es ist ferner möglich, dass der Spulenteil (erster Substratteil 110) und/oder der Bereich der elektronischen Bauelemente (dritter Substratteil 130) mit Strukturen 142 zur individuellen Codierung der Implantate versehen sind. Die Codierstrukturen können dabei derart platziert sein, dass man sie auch nach der Verkapselung und auch nach der Implantation erkennen kann. Diese Strukturen 142 zur individuellen Codierung der Implantate können beispielsweise Metall aufweisen, so dass durch ein entsprechendes Entfernen von Teilen dieser Metallstrukturen während der Montage der Bauteile jedes Implantat mit einer eindeutigen Nummer versehen werden kann.

Darüber hinaus kann die flexible Sehprothese 100 in der Nähe der Stimulationselektroden 122 oder des Stimulationselektrodenarrays 121 Längslöcher 126a oder seitliche Öffnungen 126b, 126c, 126d für die Aufnahme eines Retina-Nagels 129 in der Substratfolie aufweisen, so dass eine spätere Explantation des Implantats möglich ist, ohne dass eine Entfernung des Regina-Nagels 129 oder des Stiftes erforderlich wird.

Bei weiteren Ausführungsbeispielen sind im Bereich der Öffnungen 126 zur Aufnahme des Retina-Nagels 129 Metallstreifen 127 länglich beziehungsweise rund realisiert, deren Innendurchmesser so groß oder größer als der Retina-Nagelkopf 128 ist, so dass diese Strukturen bei der Nagelung immer sichtbar bleiben und als Orientierungshilfe für einen Chirurgen bei der Nagelung oder Befestigung verwendet werden können.

Die Metallleiterbahnen 125, die die Verbindung zwischen den elektronischen Bauelementen 132 und den Stimulationselektrodenarray 121 bilden, können galvanisch verstärkt sein, um den Ohmschen Widerstand deutlich zu verringern.

Darüber hinaus ist es möglich, dass die Stimulationselektroden 122 mit einem Material, wie beispielsweise Iridium, Iridiumoxid oder Platin/Platin black zur Erhöhung der Ladungsträgerübertragungskapazität beschichtet sind. Außerdem können die Stimulationselektroden 122 eine dreidimensionale Form aufweisen beziehungsweise dreidimensional ausgebildet sein.

Schließlich kann die Sehprothese 100 als flexible Trägerfolie und als Verkapselungsmaterial durchsichtig ausgestaltet sein, so dass zusätzlich zur elektromagnetischen Übertragung von Energie und Daten eine optische Übertragung möglich ist.

Da ein epiretinales Implantat in dieser Ausführung eine durchsichtige Verkapselung aufweist, kann zum einen ein Chirurg während der Operation durch die mittlere Öffnung 106 einen Einblick in das Innere des Auges bekommen und zum anderen die Implantatcodierung erkennen.

Somit bezieht sich die vorliegende Erfindung auf eine epiretinale Sehprothese, die erblindeten Patienten wieder eine Sehwahrnehmung ermöglichen kann. Das Ziel epiretinal implantierbarer Sehprothesen ist die elektrische Stimulation von Ganglienzellen oder anderer Teile der Netzhaut bei Patienten, bei denen es durch eine Degeneration einer vorgeschalteten retinalen Neuronen zur Erblindung gekommen ist. Derartige Situationen treten beispielsweise bei Retinitis Pigmentosa (RB), aber auch bei weit fortgeschrittenen Fällen einer Makuladegeneration auf.

Ausführungsbeispiele der vorliegenden Erfindung sind insbesondere dahingehend vorteilhaft, da sie eine sensible Behandlung der Netzhaut ermöglichen. Dies ist möglich, da durch das Falten alle schweren oder nicht-flexiblen Teile in die Linse gelegt werden können und somit der Druck auf die Netzhaut minimiert wird. Gleichzeitig bietet diese Gewichtsverlagerung in die Linse 102 auch einen verbesserte mechanischen Halt der Sehprothese.

Ein Aufkleben des Stimulators 121 auf die Netzhaut ist im Prinzip auch möglich - im Allgemeinen jedoch schwierig - weshalb in der Regel der Stimulator 121 durch Stifte beziehungsweise Nägel 129 an die Netzhaut fixiert wird. Zusätzlich zu epiretialer Stimulation ist auch eine subretinale Stimulation möglich, bei der der Stimulator zwischen verschiedenen Häute/Schichten der Netzhaut fixiert wird.

Das Substrat (Folie) kann beispielsweise Polyimid umfassen und aus mehreren Schichten bestehen (Mehrlagenfolie), wobei beispielsweise die Spule 105 innerhalb einer inneren Schicht realisiert sein kann. Bei Ausführungsbeispielen sind 25 Stimulationselektroden in den Stimulator 121 angeordnet, bei weiteren Ausführungsbeispielen können ebenfalls bis zu 100 oder bis zu 1.000 oder auch mehr Stimulationselektroden 122 in dem Stimulator 121 angeordnet sein. Die Stimulationselektroden regen dabei ganze Areale der Netzhaut an.

Die Faltung des ersten und dritten Substratteils 110, 130 oder weiterer Substratteile kann derart geschehen, dass sich Oberflächen der gefalteten Substratteile berühren oder durch eine Klebschicht in Verbindung gebracht werden. Es ist ebenfalls möglich, die Faltung durch eine mechanische Fixierung (z.B. Einklemmen, Einrasten, etc.) zu fixieren.

Zusätzlich zu den bereits beschriebenen Ausführungsbeispielen sind weitere optionale Erweiterungen möglich. Eine Möglichkeit besteht darin, dass die Sehprothese 100 einen weiteren Substratteil aufweist. Der weitere Substratteil kann beispielsweise eine weitere Spule aufweisen, die mit der Spule 105 des ersten Substratteils 110 in Reihe oder parallel geschalten ist. Das weitere Substratteil kann beispielsweise derart angeordnet sein, dass das erste Substratteil 110 zwischen dem weiteren und dem dritten Substratteil 130 angeordnet ist und somit das weitere Substratteil zusammen mit dem ersten und dem dritten Substratteil 110, 130 aufeinander faltbar sind. Bei einer Reihenschaltung der Spule 105 und der weiteren Spule wird dabei nach der Faltung effektiv eine Spule mit erhöhter Windungszahl erzeugt, währenddessen bei einer Parallelschaltung die Spulenimpedanz der effektiven Spule verkleinert wird. In gleicher Art und Weise können darüber hinaus noch zusätzliche Substratteile hinzugefügt werden, wodurch sich gleichzeitig die Induktivität der Spule deutlich erhöhen lässt.

Es kann ebenfalls vorteilhaft sein, dass vor dem Einsetzen und dem Verkleben die Sehprothese 100 zunächst außerhalb des Auges für Testzwecke verwendet wird. Das entsprechende Verfahren kann beispielsweise die folgenden Schritte umfassen: ein Erfassen eines Bildes durch die Kamera 151, ein Erzeugen eines Musters basierend auf dem erfassten Bild, ein Übertragen des Musters an die Sehprothese 100 durch die Übertragungseinrichtung 154, ein Erzeugen eines Stimulationsimpulses durch die Verarbeitungseinheit 132 und ein Weiterleiten des Stimulationsimpulses an die Stimulationselektroden 122.

Schließlich kann insbesondere für eine größere Anzahl von Stimulationselektroden 122 eine weitere vorteilhafte Ausgestaltung dahingehend durchgeführt werden, dass nicht jede Stimulationselektrode 122 einzeln über eine Verbindungsleitung 125 angesteuert wird, sondern dass eine Multiplexleitung (oder ein Bus) die Signale oder Muster für zumindest einem Teil von Stimulationselektroden 122 überträgt. Dazu kann das zweite Substratteil 120 einen Steuerchip (oder Demultiplexer) aufweisen, der die Signale von der Multiplexleitung empfängt und in Stimulationsimpulse für die einzelnen Stimulationselektroden (die Bestanteil des Teils der Stimulationselektroden 126 sind) umwandelt. Andererseits kann bei diesem Ausführungsbeispiel die Verarbeitungseinheit 132 einen Multiplexer aufweisen, der beispielsweise einen seriellen Datenstrom erzeugt, der über die Multiplexleitung transportiert werden kann.

Alternativ zu einer Multiplexleitung oder einem Datenbus können auch mehrere Multiplexleitungen ausgebildet sein, die ihrerseits jeweils einen Teil der Stimulationselektroden 122 stimulieren können. Beispielsweise können die Verbindungsleitungen 125 vier Multiplexleitungen und eine Masseleitung aufweisen. Die vier Multiplexleitungen steuern jeweils einen Teil von Stimulationselektroden 122 (z.B. zwischen 50 und 200 oder 100) mit Stimulationsimpulsen an (und stimulieren somit Abschnitte der Netzhaut 103). In diesem Fall wären dann vier Demultiplexer (oder Steurchips) in dem zweiten Substratbereich 120 nahe der Stimulationselektroden 122 angeordnet, die die Signale, die über die einzelnen Multiplexleitungen ankommen, auf die entsprechenden Stimulationselektroden 122 aufteilen.

## Patentansprüche

1. Sehprothese (100) für ein Auge (101) mit einer Linse (102) und einer Netzhaut (103), mit:
einem ersten Substratteil (110), das eine Spule (105) aufweist und in der Linse (102) fixierbar ist;
einem zweiten Substratteil (120), das Stimulationselektroden (122) und Verbindungsleitungen (125) aufweist, und an der Netzhaut (103) fixierbar ist; und
einem dritten Substratteil (130), das eine Verarbeitungseinheit (132) aufweist und das erste und das zweite Substratteil (110, 120) verbindet, wobei die Verarbeitungseinheit (132) elektrisch mit der Spule (105) und mit den Stimulationselektroden (122) verbunden und ausgelegt ist, ein in der Spule (105) durch eine elektromagnetische Welle (156a) erzeugtes Signal in einen Stimulationsimpuls umzuwandeln und an die Stimulationselektroden (122) über die Verbindungsleitung (125) weiterzuleiten,
wobei das erste Substratteil (110) bezüglich des dritten Substratteils (130) faltbar ist.

2. Sehprothese (100) nach Anspruch 1, bei der der erste Substratteil (110) zusammen mit dem dritten Substratteil (130) nach einer Faltung in der Linse (102) fixierbar ist.

3. Sehprothese (100) nach einem der vorhergehenden Ansprüche, bei der erste Substratteil (110) und der zweite Substratteil (120) ein flexibles Material aufweisen.

4. Sehprothese (100) nach einem der vorhergehenden Ansprüche, die ferner einen weiteren Substratteil aufweist, wobei der weitere Substratteil eine weitere Spule aufweist und bezüglich des ersten Substratteils (110) faltbar ist, und wobei die weitere Spule und die Spule (105) elektrisch parallel oder in Reihe geschaltet sind.

5. Sehprothese (100) nach einem der vorhergehenden Ansprüche, bei der der dritte Substratteil (130) eine ringförmige oder bevorzugt eine sichelförmige Form aufweist, so dass nach einer Faltung des ersten Substratsteils (110) bezüglich des dritten Substratteils (130) ein zentraler Bereich (106) offen bleibt.

6. Sehprothese (100) nach Anspruch 5, bei der das erste Substratteil (110) eine kreisringförmige Struktur mit einem äußeren Kreisrand (107a) und einem inneren Kreisrand (107b), der den zentralen Bereich (106) umschließt, aufweist und bei der die ringförmige oder bevorzugt die sichelförmige Form des dritten Substratteils (130) derart ausgestaltet ist, dass nach einer Faltung des ersten bezüglich des dritten Substratteils (110, 130) die kreisringförmige Struktur des ersten Substratteils (110) erhalten bleibt.

7. Sehprothese (100) nach Anspruch 6, bei der der dritte Substratteil (130) weitere ringförmige oder bevorzugt sichelförmige Abschnitte aufweist, die derart aufeinander faltbar sind, dass ein zentraler Bereich (106) des ersten Substratteils (110) offen bleibt und die kreisringförmige Struktur des ersten Substratteils (110) nach der Faltung des ersten bezüglich des dritten Substratteils (110, 130) erhalten bleibt.

8. Sehprothese (100) nach einem der vorhergehenden Ansprüche, bei der der erste Substratteil (110) nach einer Faltung mit dem dritten Substratteil (130) faltbar ist.

9. Sehprothese (100) nach einem der vorhergehenden Ansprüche, bei der der erste Substratteil (110) und der dritte Substratteil (130) nach einer Faltung mittels eines Klebstoffs aufeinander fixiert sind.

10. Sehprothese (100) nach einem der vorhergehenden Ansprüche, bei der erste Substratteil (110) eine Folienschichtstruktur aufweist, so dass die Spule (105) zwischen zwei Folienschichten angeordnet ist und hermetisch von der Umgebung abgeschlossen ist.

11. Sehprothese (100) nach einem der vorhergehenden Ansprüche, bei der erste Substratteil (110) eine zentrale Öffnung (106) aufweist, wobei die zentrale Öffnung (106) eine kreisförmige Form aufweist, durch die nach einer Fixierung in der Linse (102) eine visuelle Beobachtung der Netzhaut (103) möglich ist.

12. Sehprothese (100) nach einem der vorhergehenden Ansprüche, bei der ein Übergangsbereich zwischen dem ersten Substratteil (110) und dem dritten Substratteil (130) eine Faltunterstützungseinrichtung (210) aufweist und die Faltunterstützungseinrichtung (210) ausgelegt ist, eine Faltung entlang einer Trennlinie (200a) zu erleichtern.

13. Sehprothese (100) nach einem der vorhergehenden Ansprüche, bei der ein Übergangsbereich zwischen dem zweiten Substratteil (120) und dem dritten Substratteil (130) eine weitere Faltunterstützungseinrichtung aufweist,
wobei die weitere Faltunterstützungseinrichtung eine Faltung des dritten Substratteils (130) bezüglich des zweiten Substratteils (120) entlang einer weiteren Trennlinie (200b) zu erleichtern.

14. Sehprothese (100) nach Anspruch 12 oder Anspruch 13, bei der die Faltunterstützungseinrichtung (210) eine Einschnürung (212) und/oder eine Perforation (214) in einem Bereich zwischen dem ersten Substratteil (110) und dem dritten Substratteil (130) aufweist, und/oder
bei der die weitere Faltunterstützungseinrichtung eine Einschnürung (212) und/oder eine Perforation (214) in einem Bereich zwischen dem zweiten Substratteil (110) und dem dritten Substratteil (130) aufweist.

15. Sehprothese (100) nach einem der vorhergehenden Ansprüche, bei der das erste Substratteil (110) eine kreisringförmige Struktur mit einem äußeren Kreisrand (107a) und einem inneren Kreisrand (107b) aufweist, wobei entlang des inneren Kreisrandes (107b) eine erste Justagehilfe (144a) ausgebildet ist,
und wobei der dritte Substratteil (130) eine sichelförmige Form aufweist, mit einem äußeren Sichelkreis (307a) und einem inneren Sichelkreis (307b), wobei entlang des inneren Sichelkreises (307b) eine weitere Justagehilfe (144b) ausgebildet ist,
wobei die Justagehilfe (144a) und die weitere Justagehilfe (144b) derart ausgebildet sind, so dass bei einer Faltung des ersten bezüglich des dritten Substratteils (110, 130) die Justagehilfe (144a) und die weitere Justagehilfe (144b) aufeinander angeordnet sind.

16. Sehprothese (100) nach einem der vorhergehenden Ansprüche, die Metallstrukturen aufweist, wobei die Metallstrukturen eine individuelle Codierung durch ein Entfernen von Teilen der Metallstrukturen ermöglichen.

17. Sehprothese (100) nach Anspruch 16, bei der die Metallstrukturen zur Codierung derart angeordnet sind, dass nach einer Implantation der Sehprothese (100) ins Auge die Metallstrukturen sichtbar bleiben.

18. Sehprothese (100) nach einem der vorhergehenden Ansprüche, bei der der zweite Substratteil (120) Befestigungshilfen (126) aufweist, wobei die Befestigungshilfen (126) ausgelegt sind, um eine mit der Netzhaut (103) verbundene weitere Justierhilfe (129) in Eingriff zu nehmen.

19. Sehprothese (100) nach Anspruch 18, bei der die Befestigungshilfe (126) ein Längsloch (126a) und/oder ein Loch mit einer seitlichen Öffnung (126b; 126c; 126d) aufweist, so dass ein Entfernen des Stimulators (121) ohne ein Entfernen der weiteren Justierhilfe (129) möglich ist.

20. Sehprothese (100) nach Anspruch 18 oder Anspruch 19, bei der die weitere Justierhilfe (129) einen RetinaNagel aufweist.

21. Sehprothese (100) nach einem der Ansprüche 18 bis 20, bei der die Befestigungshilfe (126) ferner eine Positionshilfe (127) aufweist, wobei die Positionshilfe (127) ausgebildet ist, um eine relative Positionierung des Stimulators (121) in Bezug auf die weitere Justierhilfe (129) zu ermöglichen.

22. Sehprothese (100) nach einem der vorhergehenden Ansprüche, bei der die Verbindungsleitungen (125) einen Querschnitt aufweisen, so dass ein Ohmscher Widerstand während einer Stimulation der Netzhaut (103) unterhalb eines vorbestimmten Werts liegt.

23. Sehprothese (100) nach einem der vorhergehenden Ansprüche, bei der die Stimulationselektrode (122) um eine Höhe (H) aus einer Oberfläche (123) des Stimulators (121) herausragen und die Höhe (H) einen vorbestimmten Minimalwert überschreitet.

24. Sehprothese (100) nach Anspruch 23, bei der der vorbestimmte Minimalwert 10 µm oder 30 µm aufweist.

25. Sehprothese (100) nach einem der vorhergehenden Ansprüche, bei der die Stimulationselektroden (122) eine Beschichtung aufweisen, die ausgelegt ist, um einen Übergangswiderstand zwischen den Stimulationselektroden (122) und der Netzhaut (103) zu verringern.

26. Sehprothese (100) nach Anspruch 25, bei der die Beschichtung Iridium, Iridiumoxid oder Platin/Platin black aufweist.

27. Sehprothese (100) nach einem der vorhergehenden Ansprüche, bei der die Verbindungsleitung (125) eine Multiplexleitung aufweist und der zweite Substratteil (120) einen Steuerchip aufweist,
wobei der Steuerchip ausgebildet ist, Signale von der Multiplexleitung zu empfangen und basierend auf den Signalen Stimulationsimpulse zumindest an einen Teil der Stimulationselektroden (126) zu senden.

28. Sehprothese (100) nach einem der vorhergehenden Ansprüche, bei der der erste Substratteil (110) und der dritte Substratteil (130) eine Folienbeschichtung aufweisen, die transparent für eine elektromagnetische und/oder optische Übertragung ist.

29. Sehprothese (100) für ein Auge (101) mit einer Linse (102) und einer Netzhaut (103), mit:
einem ersten Substratteil (110), das eine Spule (105) aufweist und in der Linse (102) fixierbar ist;
einem zweiten Substratteil (120), das Stimulationselektroden (122) und Verbindungsleitungen (125) aufweist und an der Netzhaut (103) fixierbar ist; und
einem dritten Substratteil (130), das eine Verarbeitungseinheit (132) aufweist, und das erste und das zweite Substratteil (120) verbindet, wobei die Verarbeitungseinheit (132) elektrisch mit der Spule (105) und den Stimulationselektroden (122) verbunden und ausgelegt ist, ein in der Spule (105) durch eine elektromagnetische Welle (156a) erzeugte Signal in Stimulationsimpulse umzuwandeln und an die Verbindungsleitung (125) weiterzuleiten,
wobei der zweite Substratteil (120) eine Befestigungshilfe (126) aufweist, die ausgelegt ist, um eine mit der Netzhaut (103) verbundene weitere Justierhilfe in Eingriff zu nehmen.

30. Verfahren zur Verwendung einer Sehprothese (100) nach Anspruch 1 oder Anspruch 29, mit:
Erfassen eines Bildes durch eine Kamera (151);
Erzeugen eines Musters von dem erfasste Bild;
Übertragen des Musters an die Sehprothese (100) durch eine Übertragungseinrichtung (154);
Erzeugen eines Stimulationsimpulses durch die Verarbeitungseinheit (132); und
Weiterleiten des Stimulationsimpulses an die Stimulationselektroden (122).

31. Verfahren zur Herstellung einer Sehprothese (100) für ein Auge (101) mit einer Linse (102) und einer Netzhaut (103), mit folgenden Schritten:
Bereitstellen eines ersten Substratteils (110), wobei das erste Substratteil (110) eine Spule (105) aufweist und in der Linse (102) fixierbar ist;
Bereitstellen eines zweiten Substratteils (120), das Stimulationselektroden (122) und Verbindungsleitungen (125) aufweist und an der Netzhaut (103) fixierbar ist; und
Verbinden des ersten Substratteils (110) und des zweiten Substratteils (120) durch ein drittes Substratteil (130), wobei das dritte Substratteil (130) eine Verarbeitungseinheit (132) aufweist, so dass die Spule (105) mit den Stimulationselektroden (122) verbunden wird, so dass ein in der Spule (105) durch eine elektromagnetische Welle (156a) erzeugte Signal in ein Stimulationsimpulse umgewandelt wird und an die Verbindungsleitungen (125) weitergeleitet wird,
wobei das dritte Substratteil (130) mit dem ersten Substratteil (110) faltbar miteinander verbunden werden.

32. Verfahren zum Anbringen einer Sehprothese (100), die einen ersten, zweiten und dritten Substratteil (110, 120, 130) aufweist, an einer vorbestimmten Position in einem Auge (101) mit einer Linse (102) und einer Netzhaut (103), mit folgenden Schritten:
Falten des ersten Substratteils (110) bezüglich des dritten Substratteils (130);
Verfüllen des gefalteten ersten und dritten Substratteils (110, 130) mit Silikon;
Falten des verfüllten ersten und dritten Substratteils (110, 130) entlang ein Linie, die parallel zu einer Einbringrichtung der Sehprothese (100) in das Auge (101) verläuft;
Einbringen der Sehprothese (100) in das Auge (101);
Fixieren des gefalteten ersten und dritten Substratteils (110, 130) in der Linse (102); und
Fixieren eines zweiten Substratteils (120), das Stimulationselektroden (122) und eine Verbindungsleitung (125) aufweist, an der Netzhaut (103).
